(19)

Europäisches
Patentamt
European
Patent Office
Office européen
des brevets

(11) **EP 4 596 722 A1**

(12) **EUROPEAN PATENT APPLICATION**
published in accordance with Art. 153(4) EPC

(43) Date of publication:
06.08.2025 Bulletin 2025/32

(21) Application number: 23907212.7

(22) Date of filing: 22.12.2023

(51) International Patent Classification (IPC):
C21B 5/06 (2006.01)    C07C 1/04 (2006.01)
C07C 1/12 (2006.01)    C10K 3/06 (2006.01)
C21B 5/00 (2006.01)    C21B 13/02 (2006.01)
F27D 17/00 (2025.01)

(52) Cooperative Patent Classification (CPC):
C07C 1/04; C07C 1/12; C10K 3/06; C21B 5/00;
C21B 5/06; C21B 13/02; F27D 17/00; Y02P 10/143

(86) International application number:
PCT/JP2023/046273

(87) International publication number:
WO 2024/135852 (27.06.2024 Gazette 2024/26)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 23.12.2022 JP 2022207619

(71) Applicant: JFE Steel Corporation
Tokyo 100-0011 (JP)

(72) Inventors:
• NAKAHARA, Yoshiko
Tokyo 100-0011 (JP)
• MORIYA, Kota
Tokyo 100-0011 (JP)
• TERUI, Koki
Tokyo 100-0011 (JP)
• NOUCHI, Taihei
Tokyo 100-0011 (JP)

(74) Representative: Scott, Stephen John
YUJA IP LAW
4 Centenary House
The Avenue
York YO30 6AU (GB)

(54) **REDUCED IRON PRODUCTION METHOD**

(57)    Provided is a method of producing reduced iron that can achieve both energy savings and a decrease in $CO_2$ emissions. The system is configured to circulate and reuse top gas with a blowing process, a reduction process, a distribution process, a synthesis process, and a reforming process, and the distribution ratio of the top gas in the distribution process is controlled according to consumed $H_2/CO$, which is a ratio of the amount of $H_2$ consumed to the amount of CO consumed in the reduction process.

FIG. 2

EP 4 596 722 A1

## Description

TECHNICAL FIELD

The present disclosure relates to a method of producing reduced iron.

BACKGROUND

In recent years, there has been a strong demand for energy savings in steelworks against the backdrop of global environmental issues and fossil fuel depletion issues. The raw material of iron is mainly iron oxide, and a reduction process to reduce this iron oxide is essential in steelworks. The most widespread and common reduction process worldwide uses a blast furnace. In a blast furnace, coke or pulverized coal reacts with oxygen in hot blast (air heated to about 1200 °C) from a tuyere. This reaction produces CO and $H_2$ as reducing gases, which are used to reduce the iron ore and the like in the furnace. Recent improvements in blast furnace operation technology have decreased the reducing agent rate (the amount of coke and pulverized coal used per tonne of hot metal produced) to about 500 kg/t, and the reducing agent rate has already almost reached a lower limit. Therefore, no further significant decrease in the reducing agent rate is expected.

On the other hand, in regions where natural gas is produced, a vertical reduction furnace (hereinafter also referred to as a shaft furnace) is often used to produce reduced iron. In this method, the reduction furnace is charged with sintered ore, pellets, or other agglomerated iron ore as the iron oxide raw material (hereinafter also simply referred to as iron oxide). Reducing gas including CO and $H_2$ is then blown into the reduction furnace to reduce the iron oxide to produce reduced iron. In this method, natural gas or the like is used as the feed gas for the reducing gas. This feed gas is heated and reformed together with top gas in a reformer. This produces reducing gas. Here, the top gas is gas after the iron oxide is reduced in the reduction furnace, and is typically discharged from the top of the reduction furnace. The reducing gas is blown into the reduction furnace and reacts with iron oxide supplied from the top of the furnace. The iron oxide is then reduced to reduced iron. The reduced iron is then cooled in a region below where the reducing gas is blown into the reduction furnace, and then discharged from the bottom of the reduction furnace.

Further, as mentioned above, the top gas, which is gas after iron oxide is reduced, is discharged from the reduction furnace, for example, from the top of the furnace. After dust collection and cooling is applied to the top gas, some is fed to the reformer as raw material for reformed gas. Further, remaining top gas is used as fuel gas for the reformer. In this method, the top gas used as fuel gas for the reformer is normally discharged out of the system.

As such a reduced iron production process, for example, Patent Literature (PTL) 1 describes a method of producing reduced iron by reforming exhaust gas from a reduction furnace and natural gas in a reformer to produce reducing gas consisting mainly of CO and $H_2$, blowing the reducing gas into the reduction furnace, and reducing iron oxide in the reduction furnace.

Further, PTL 2 describes a method of producing reduced iron by reforming coke oven gas and $CO_2$-removed top gas from a reduction furnace to produce reducing gas, which is then blown into a reduction furnace.

CITATION LIST

Patent Literature

PTL 1: JP 2017-088912 A
PTL 2: JP 6190522 B2

SUMMARY

(Technical Problem)

The method described in PTL 1 uses externally supplied natural gas for the production of reducing gas. Therefore, although lower than that of blast furnaces, there is a problem that a certain amount of $CO_2$ emissions are unavoidable.

Further, the method described in PTL 2 uses coke oven gas or converter gas produced in a steelworks to produce reducing gas. Here, in an integrated steelworks, coke oven gas and converter gas are needed as fuel gas for downstream processes such as a heating furnace and an annealing furnace. Therefore, when coke oven gas or converter gas is diverted to the reduced iron production process, a fuel gas shortage is caused in the downstream processes. As a result, natural gas is supplied from outside to compensate for the shortage of fuel gas in the downstream processes. That is, even with the method described in PTL 2, energy savings and a decrease in $CO_2$ emissions could not both be achieved, and the problem remained.

In view of the circumstances described, it would be helpful to provide a method of producing reduced iron that can realize both energy savings and a decrease in $CO_2$ emissions.

(Solution to Problem)

The inventors have conducted extensive research to realize both energy savings and a decrease in $CO_2$ emissions, and developed a system that circulates and reuses top gas.

That is, the inventors developed

a top gas circulation and reuse system (hereinafter also simply referred to a circulation system) comprising:

a blowing process of blowing reducing gas into a reduction furnace;
a reduction process, in the reduction furnace, of reducing iron oxide by the reducing gas to obtain reduced iron;
a synthesis process of synthesizing regenerative methane gas from top gas discharged from the reduction furnace and hydrogen gas; and
a reforming process of using the regenerative methane gas as feed gas to obtain reducing gas from the feed gas.

The inventors further investigated and made the following discoveries.

- In order to stably carry out an operation that achieves both energy savings and a decrease in $CO_2$ emissions, it is important to maintain a healthy mass balance, in particular the mass balance of C (carbon), in the circulation system described above.
- For this purpose, it is important to control the gas balance of the circulation system so that the ratio of the amount of $H_2$ to the amount of CO in the reducing gas supplied to the reduction furnace (hereinafter also referred to as $H_2/CO$ of the reducing gas) is always maintained within a certain range without significant fluctuations. The $H_2/CO$ of the reducing gas is the volume ratio under standard conditions (which may also be called the flow rate ratio under standard conditions). The same is true for consumed $H_2/CO$ and the like, as described below.

There is an optimum energy range for the $H_2/CO$ of the reducing gas, depending on furnace temperature and pressure in a direct reduction furnace and furnace size. Further, as examples of raw material for the reducing gas, in addition to natural gas used in a typical direct reduction furnace such as Midrex® (Midrex is a registered trademark in Japan, other countries, or both) and Hyl® (Hyl is a registered trademark in Japan, other countries, or both), coke oven gas, hydrocarbon, waste plastic, and biomass combustion gas may be used. When these gases are used, the $H_2/CO$ of the reducing gas also changes to various values depending on composition. The direct reduction furnaces currently in operation are actually operating in a range of the $H_2/CO$ of the reducing gas appropriate for their respective plants. However, the composition of top gas may change when the grade of iron oxide, the raw material of reduced iron, changes or when furnace temperature changes. In such cases, the $H_2/CO$ of the reducing gas also changes, making it difficult to maintain a healthy mass balance in the circulation system described above.

Regarding the above points, the inventors made further studies and found that the following controls make it possible to maintain a healthy mass balance in the circulation system, that is, stably carry out an operation that achieves both energy savings and a decrease in $CO_2$ emissions during the production of reduced iron.

- Top gas discharged from the reduction furnace is distributed into a first top gas and a second top gas.
- The distribution ratio of the top gas in the distribution process is controlled according to the ratio of the amount of $H_2$ consumed to the amount of CO consumed in the reduction process, that is, consumed $H_2/CO$.

The present disclosure is based on these discoveries and further studies.

Primary features of the present disclosure are as follows.

1. A method of producing reduced iron, the method comprising:

a charging process of charging iron oxide into a reduction furnace;
a blowing process of blowing reducing gas into the reduction furnace;
a reduction process, in the reduction furnace, of reducing the iron oxide by the reducing gas to obtain reduced iron;
a distribution process of distributing top gas discharged from the reduction furnace into a first top gas and a second top gas;
a synthesis process of synthesizing regenerative methane gas from the first top gas and hydrogen gas; and

a reforming process of using the regenerative methane gas and the second top gas as feed gas to obtain the reducing gas from the feed gas, wherein

a distribution ratio of the top gas in the distribution process is controlled according to consumed $H_2/CO$, a ratio of an amount of $H_2$ consumed to an amount of CO consumed in the reduction process.

2. The method of producing reduced iron according to 1, above, the method further comprising a separation process between the distribution process and the synthesis process, of separating the first top gas into carbon dioxide gas and remaining gas, wherein

the carbon dioxide gas is used as the first top gas in the synthesis process.

3. The method of producing reduced iron according to 1 or 2, above, wherein a distribution ratio, in %, of $Y \times 100$ to the first top gas in the distribution process is controlled in a range of $Y_0 \times 100 \pm 5\%$, $Y_0 \times 100$ being a standard distribution ratio, in %,

where

when

$$X \leq 1, \ Y_0 = 1/(2\eta)$$

when

$$1 < X < 4\eta - 1, \ Y_0 = (X + 1)/(4\eta)$$

when

$$X \geq 4\eta - 1, \ Y_0 = 1$$

and

X is consumed $H_2/CO$

$\eta \times 100$ is $CO_2$ conversion rate, in %, from methane synthesis in the synthesis process

and when the range of $Y_0 \times 100 \pm 5\%$ exceeds 100 %, an upper limit of the range is 100 %.

4. The method of producing reduced iron according to 3, above, wherein the distribution ratio, in %, of $Y \times 100$ to the first top gas in the distribution process is controlled to $Y_0 \times 100$ being the standard distribution ratio, in %.

5. The method of producing reduced iron according to 3 or 4, above, wherein excess $H_2$ is recovered from a circulation system comprising the blowing process, the reduction process, the distribution process, the synthesis process, and the reforming process when the consumed $H_2/CO < 1$.

6. The method of producing reduced iron according to 3 or 4, above, wherein $H_2$ is additionally supplied to a circulation system comprising the blowing process, the reduction process, the distribution process, the synthesis process, and the reforming process when the consumed $H_2/CO > 4\eta - 1$.

7. The method of producing reduced iron according to any one of 1 to 6, above, wherein at least one of de-dusting or dehydration of the top gas is carried out prior to the distribution process.

8. The method of producing reduced iron according to any one of 1 to 7, above, wherein dehydration of the regenerative methane gas is carried out prior to the reforming process.

(Advantageous Effect)

According to the present disclosure, it is possible to stably carry out an operation that achieves both energy savings and a decrease in $CO_2$ emissions during the production of reduced iron.

BRIEF DESCRIPTION OF THE DRAWINGS

In the accompanying drawings:

FIG. 1 is a diagram illustrating a conventional reduced iron production process;

FIG. 2 is a diagram illustrating an example of a reduced iron production process according to a method of producing reduced iron according to an embodiment of the present disclosure; and

FIG. 3 is a diagram illustrating the relationship between consumed $H_2/CO$:X and distribution ratio Y of a first top gas.

## DETAILED DESCRIPTION

The following is a description of a method of producing reduced iron according to an embodiment of the present disclosure, with reference to the drawings.

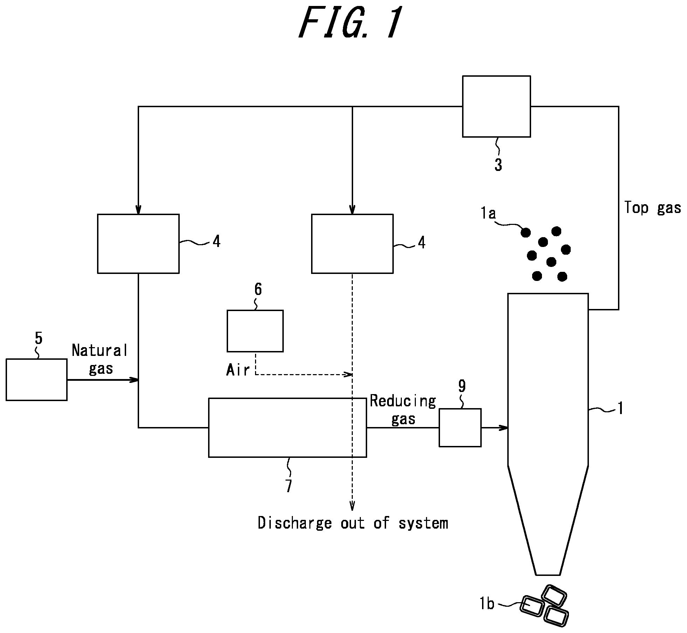

First, a conventional reduced iron production process (hereinafter also referred to as a conventional production process) is described. FIG. 1 is a schematic diagram illustrating an example of a conventional production process. In the drawing, reference sign 1 is a reduction furnace, 1a is iron oxide, 1b is reduced iron, 3 is a deduster, 4 is a dehydrator, 5 is a natural gas supply, 6 is an air supply, 7 is a reformer, and 9 is a reducing gas blowing device.

In the example of a conventional production process, iron oxide is charged from the top of the reduction furnace and gradually descends. There, iron oxide is reduced by blowing in high-temperature reducing gas from a central portion of the reduction furnace. The reduced iron is then discharged from the bottom of the reduction furnace. In this process, top gas containing mainly CO, $CO_2$, $H_2$, and $H_2O$ is discharged from the top of the reduction furnace. The top gas is de-dusted by the deduster, and a portion is subjected to moisture adjustment and fed to the reformer as feed gas. Gas containing hydrocarbons, for example, natural gas from a natural gas supply, is supplied to the reformer along with the top gas that has been subjected to moisture adjustment. The supplied gas is then heated in the reformer. A reforming reaction then occurs, producing high-temperature reducing gas containing mainly CO and $H_2$. This reducing gas is then blown into the reduction furnace. Further, the remaining portion of the top gas is dehydrated and used as fuel for heating in the combustion chamber of the reformer, for example, by combustion with oxygen in the air. After combustion as fuel for heating, the top gas still containing $CO_2$ is normally discharged out of the system.

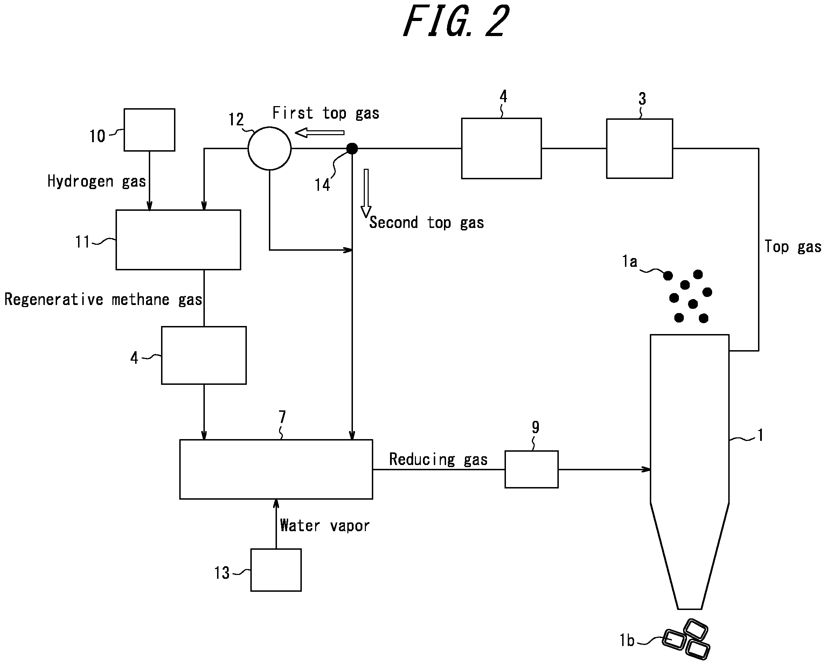

In contrast, in the method of producing reduced iron according to an embodiment of the present disclosure, as illustrated in FIG. 2 for example, the top gas discharged from the reduction furnace is distributed into first top gas and second top gas in a top gas distributor. Methane gas synthesized from the first top gas and hydrogen gas (hereinafter also referred to as regenerative methane gas) is used instead of hydrocarbon gas such as natural gas supplied from outside, as in the conventional process illustrated in FIG. 1. In the drawing, reference sign 10 is a hydrogen supply, 11 is a methane synthesizer, 12 is a $CO_2$ separator, 13 is a water vapor supply, and 14 is a top gas distributor. Here, the regenerative methane gas produced in the methane synthesizer 11 is supplied to the reformer 7 to be used as feed gas for the reducing gas.

The following is a description of each process of the method of producing reduced iron according to an embodiment of the present disclosure. The charging process, blowing process, and reduction process can be carried out in accordance with conventional methods, for example, in the same manner as in the conventional process described above, and therefore description is omitted here.

• Distribution Process

In the distribution process, for example, the top gas discharged from the reduction furnace is distributed into the first top gas and the second top gas in the top gas distributor. In this case, it is important to control a distribution ratio to the first top gas according to consumed $H_2$/CO, which is the ratio of the amount of $H_2$ consumed to the amount of CO consumed in the reduction process. The reasons for this are discussed below. Further, distribution of the top gas and a flow rate controller are not particularly limited and may follow a conventional method. For example, a mass flow controller or the like may be used.

• Separation process

Between the distribution process described above and the synthesis process described below, a separation process may be further included, of separating the first top gas into carbon dioxide gas and remaining gas by, for example, a $CO_2$ separator. The method of $CO_2$ separation is not particularly limited, and various methods may be used, examples including chemical absorption, physical absorption, adsorption separation, membrane separation, deep cold separation, oxygen combustion, chemical loop combustion, and the like. Among these, in particular, chemical absorption methods, of which an amine absorption method is representative, and adsorption separation methods, of which a PSA method is representative, have long been used in chemical plants, industrial $CO_2$ production, and the like. The $CO_2$ concentration in the carbon dioxide gas is preferably 90 vol% or more. The $CO_2$ concentration in the carbon dioxide gas may be 100 vol%. Further, the remaining gas may, for example, be introduced into the reforming process described below, together with the second top gas.

• Synthesis process

In the synthesis process, for example, regenerative methane gas is synthesized in a methane synthesizer from the first top gas distributed by the distribution process described above and hydrogen gas. Further, in the case of the separation process described above, carbon dioxide gas is suitable to be used as the first top gas in this process. $CH_4$ is synthesized from at least one of $CO_2$ or $CO$ in the first top gas and $H_2$ according to the following methanation reaction equations in Expressions (i) and (ii).

$$CO_2 + 4H_2 \rightarrow CH_4 + 2H_2O \ \Delta H = \text{-165 kJ/mol} \ \dots \qquad (i)$$

$$CO + 3H_2 \rightarrow CH_4 + H_2O \ \Delta H = \text{-206 kJ/mol} \ \dots \qquad (ii)$$

For example, the first top gas and hydrogen gas supplied from outside the circulation system described above are introduced into the methane synthesizer. $CH_4$ is then synthesized in the methane synthesizer by at least one of the reactions in Expressions (i) and (ii). Conditions for $CH_4$ synthesis are not particularly limited, and a conventional method may be followed.

When the carbon dioxide gas obtained in the separation process described above is used, the composition of the first top gas is the same as that of the carbon dioxide gas. Further, when the separation process described above is not carried out, the composition of the first top gas is basically the same as the composition of the top gas. In such a case, the composition of the first top gas is CO: 5 vol% to 50 vol%, $CO_2$: 5 vol% to 30 vol%, $H_2$: 5 vol% to 80 vol%, $H_2O$: 0 vol% to 35 vol%, with the balance being: 0 vol% to 20 vol%. The composition of the second top gas, described below, is similar.

In addition to the first top gas, any other gas containing at least one of CO or $CO_2$ (hereinafter also referred to as "other gas") may be used in the synthesis process. Examples of the other gas include by-product gas of a steelmaking process, specifically blast furnace gas (BFG) and coke oven gas (COG). Further, the other gas may be introduced into the separation process described above along with the first top gas and separated into carbon dioxide gas and remaining gas before supplying the carbon dioxide gas to the synthesis process.

Further, the source of hydrogen gas used in the synthesis process is not particularly limited and the hydrogen gas may be supplied and produced by any method. Methods of producing hydrogen gas include, for example, synthesis by electrolysis of water and by decomposition reactions of ammonia, hydrocarbons, and organic hydrides. However, when hydrocarbons or organic hydrides are used as a raw material, $CO_2$ is emitted in the hydrogen synthesis process. Therefore, from the viewpoint of further decreasing $CO_2$ emissions, synthesis by at least one of the electrolysis of water or decomposition of ammonia is preferred. Further, when hydrogen gas is produced by electrolysis of water, green hydrogen produced using electrical power from green energy sources such as solar, wind, and geothermal power may be used to decrease $CO_2$ emissions to zero. The $H_2$ concentration of the hydrogen gas is not particularly limited. The $H_2$ concentration of the hydrogen gas is preferably 90 vol% or more. The $H_2$ concentration of the hydrogen gas is more preferably 95 vol% or more. The $H_2$ concentration of the hydrogen gas may be 100 vol%.

Further, the amount of hydrogen gas supplied in the synthesis process (the amount of hydrogen gas supplied to the methane synthesizer) is preferably a stoichiometric amount relative to the amount of $CO_2$ contained in the first top gas, based on the methanation reaction equation in Expression (i).

In the synthesis of $CH_4$, a typically used methanation catalyst may be used. Specifically, a transition metal catalyst such as Fe, Ni, Co, Ru, or the like may be used. Among these, Ni catalysts are the most active. Further, Ni catalysts also have high heat resistance and are usable up to about 500 °C. Therefore, Ni catalysts are particularly preferred. Further, iron ore may be used as a catalyst. Ores containing high crystallization water are particularly suitable for use as catalysts because the specific surface area increases when the water of crystallization is dehydrated.

The reactor of the methane synthesizer used in the synthesis process may be a fixed bed reactor, a fluidized bed reactor, an air flow bed reactor, or the like. Depending on the form of these reactors, the physical properties of the catalyst may be selected accordingly. Further, a heat exchanger may be disposed in the gas flow path downstream of the reactor to recover heat from the reaction heat (gas sensible heat) of the methanation reaction in each reactor. The recovered thermal energy may, for example, be used to heat the reduction furnace or the reformer.

Further, the methanation catalysts described above exhibit a stable high conversion rate during $CO_2$ methanation. However, there have been reported cases of catalyst poisoning during CO methanation due to C precipitation caused by the formation of C intermediates. Therefore, it is preferable to have a separation process between the distribution process and the synthesis process, where the first top gas is separated into carbon dioxide gas and remaining gas. In other words, the carbon dioxide gas separated from the first top gas in the separation process is used as the first top gas in the synthesis process.

When $H_2O$ produced as a byproduct of methane synthesis is introduced into the reformer, there may be an excess of $H_2O$ in the reforming process described below. Therefore, while considering the mass balance of the circulation system as a whole, it is preferable to dehydrate the regenerative methane gas using a dehydrator, as appropriate, prior to the reforming process described below.

The $CH_4$ concentration of the regenerative methane gas is not particularly limited. In regenerative methane gas from which $H_2O$ is removed, that is, in regenerative methane gas after dehydration of the gas leaving the methane synthesizer by a dehydrator, the $CH_4$ concentration is preferably 80 vol% or more. The $CH_4$ concentration is more

preferably 90 vol% or more. The $CH_4$ concentration in regenerative methane gas without $H_2O$ may be 100 vol%.

• Reforming process

In the reforming process, the regenerative methane gas and the second top gas are used as feed gas to obtain reducing gas from the feed gas. For example, the regenerative methane gas and the second top gas are introduced into the reformer, and the regenerative methane gas and the second top gas are heated in the reformer. Then, in the reformer, the reforming reactions of the following Expressions (iii) and (iv) are used to produce reducing gas containing CO and $H_2$. By supplying water vapor to the reformer, the reforming reaction represented in Expression (iv) proceeds.

$$CH_4 + CO_2 \rightarrow 2CO + 2H_2 \ \Delta H = 247 \ kJ/mol \ ... \quad (iii)$$

$$CH_4 + H_2O \rightarrow CO + 3H_2 \ \Delta H = 206 \ kJ/mol \ ... \quad (iv)$$

The gas composition of the reducing gas is, for example, CO: 1 vol% to 60 vol%, $H_2$: 40 vol% to 99 vol%, with the balance being 0 vol% to 30 vol%.

As indicated by the positive enthalpies of formation in Expressions (iii) and (iv), both reactions in Expressions (iii) and (iv) are endothermic reactions. Therefore, by using green energy sources such as solar power, wind, geothermal energy, and the like as an energy source during the reforming reaction, $CO_2$ emissions may in principle be decreased to zero.

The reducing gas is then introduced into the reduction furnace through a blowing process. For example, the reducing gas is introduced into the reduction furnace using a reducing gas blowing device. The iron oxide is then reduced by the reducing gas in the reduction furnace to obtain reduced iron. On the other hand, the reducing gas after being used for iron oxide reduction is discharged from the reduction furnace as the top gas.

Further, it is preferred that at least one of de-dusting or dehydration of the top gas is carried out prior to the distribution process. Any deduster may be used for de-dusting. Further, any dehydrator may be used for dehydration. The order of dedusting and dehydration is not particularly limited. In the example illustrated in FIG. 2, the top gas is dehydrated by the dehydrator after de-dusting by the deduster, and then the top gas is distributed into the first top gas and the second top gas.

• Top gas distribution ratio in distribution process

In the method of producing reduced iron according to an embodiment of the present disclosure, it is important to control the distribution ratio of the top gas in the distribution process according to the consumed $H_2/CO$, which is the ratio of the amount of $H_2$ consumed to the amount of CO consumed in the reduction process (hereinafter also simply referred to as consumed $H_2/CO$).

Here, the consumed $H_2/CO$ is calculated, for example, by the following expression.

consumed $H_2/CO$ = ([amount of $H_2$ contained in reducing gas blown --> into reduction furnace ($Nm^3/t$)] - [amount of $H_2$ contained in top gas ($Nm^3/t$)]) / ([amount of CO contained in reducing gas blown into reduction furnace ($Nm^3/t$)] - [amount of CO contained in top gas ($Nm^3/t$)])

Here, $Nm^3/t$ is the basic unit per tonne of reduced iron (DRI) produced.

For example, the distribution ratio of $Y \times 100$ (%) to the first top gas is controlled around the standard distribution ratio of $Y_0 \times 100$ (%), which is calculated according to X. For example, $Y \times 100$ (%) is preferably controlled in the range of $Y_0 \times 100$ (%) $\pm 5$ %. $Y \times 100$ (%) is more preferably in the range of $Y_0 \times 100$ (%) $\pm 3$ %. $Y \times 100$ (%) is even more preferably in the range of $Y_0 \times 100$ (%) $\pm 1\%$. The distribution ratio of $Y \times 100$ (%) to the first top gas is most preferably controlled to $Y_0 \times 100$ (%).

Here,

when

$$X \leq 1, \ Y_0 = 1/(2\eta),$$

when

$$1 < X < 4\eta - 1, Y_0 = (X + 1)/(4\eta),$$

and
when

$$X \geq 4\eta - 1, Y_0 = 1.$$

Further,

X is consumed $H_2/CO$, and
$\eta \times 100$ is $CO_2$ conversion rate from methane synthesis in the synthesis process (%).

Further, when the ranges of $Y_0 \times 100 \pm 5$ %, $Y_0 \times 100 \pm 3$ %, and $Y_0 \times 100 \pm 1$% each exceed 100 %, an upper limit of the ranges is 100 %.

Y is defined by the following expression.

Y = [amount of top gas distributed to first top gas in distribution process ($Nm^3/t$)] ÷ [amount of top gas introduced into distribution process ($Nm^3/t$)]

The distribution ratio to the second top gas is also defined in the same way as in the above expression. The top gas is basically distributed into the first top gas and the second top gas, and therefore the distribution ratio to the second top gas is also appropriately controlled by appropriate control of the distribution ratio to the first top gas. Further, in this case, the distribution ratio to the second top gas is 1 - Y. In addition, a portion of the top gas may be supplied to other loads depending on operating conditions and other factors, as long as the portion is 10 vol% or less of the top gas volume. The amount of the top gas supplied to other loads is not included in the amount of the top gas introduced into the distribution process.

Further, the $CO_2$ conversion rate $\eta \times 100$ (%) from methane synthesis in the synthesis process is defined by the following expression.

[$CO_2$ conversion rate $\eta \times 100$ (%)] = (1 - [amount of $CO_2$ contained in regenerative methane gas emitted after synthesis of $CH_4$ in synthesis process ($Nm^3/t$)] ÷ [amount of $CO_2$ introduced into synthesis process ($Nm^3/t$)]) $\times$ 100

The following describes the standard distribution ratio of $Y_0 \times 100$ (%). First, when iron oxide is reduced by a reducing gas that is a mixed gas of $H_2$ and CO, the consumption of $H_2$ and CO is determined by the equilibrium and reaction rate constants in Expressions (v) and (vi) below. For example, when the grade of the raw iron ore decreases, the activation energy of $H_2$ reduction increases, which is assumed to reduce the progression of Expression (vi). Thus, the amount of $H_2$ and the amount of CO consumed in reduction varies in various ways depending on operating conditions.

$$3CO + Fe_2O_3 \rightarrow 3CO_2 + 2Fe \text{ ...} \qquad \text{(v)}$$

$$3H_2 + Fe_2O_3 \rightarrow 3H_2O + 2Fe \text{ ...} \qquad \text{(vi)}$$

In the method of producing reduced iron according to an embodiment of the present disclosure, $H_2$ and CO equivalent to the amounts consumed by reduction are synthesized by reforming $CH_4$ to compensate for the consumption, thereby making it possible to keep the composition of the reducing gas blown into the reduction furnace constant. That is, in Expression (v), the amount of CO consumed by the reduction is equivalent to the amount of $CO_2$ produced by the reduction. A portion of this $CO_2$ is then converted to methane in the synthesis process as the first top gas, and then supplied to the reforming process. Further, the remaining $CO_2$ is supplied to the reforming process without going through the synthesis process as the second top gas. That is, focusing on mass balance for C overall, in the circulation system described above, the amount of CO consumed by the reduction of iron oxide in the reduction process is practically equivalent to the amount of CO produced in the reforming process.

Here, as indicated in Expressions (iii) and (iv), when $CH_4$ is reformed by $CO_2$, $H_2$ and CO are produced in a 1:1 ratio. On the other hand, when $CH_4$ is reformed by $H_2O$, $H_2$ and CO are produced in a ratio of 3:1. The distribution ratio to the first top gas is synonymous with the ratio of $CO_2$ in the top gas that is supplied to the synthesis process. The distribution ratio to the second top gas is synonymous with the ratio of $CO_2$ in the top gas that is supplied to the reforming process. In other words, the distribution ratio to the second top gas supplied to the reforming process can be said to represent the ratio of the reforming reaction due to $CO_2$ in Expression (iii). Therefore, the distribution ratio of the top gas is an important factor in maintaining a healthy mass balance in the circulation system, in particular the mass balance of C overall.

When the total carbon equivalent of $CO_2$ contained in the top gas introduced into the distribution process is set to 1, the carbon equivalent of $CH_4$ in the regenerative methane gas synthesized in the synthesis process is expressed as $Y\eta$, which is the distribution ratio Y of the first top gas multiplied by the $CO_2$ conversion ratio $\eta$ from methane synthesis in the synthesis process.

Further, the $CO_2$ introduced into the reforming process is the sum of the $CO_2$ contained in the second top gas and the $CO_2$ remaining in the first top gas that is not synthesized into methane in the synthesis process. Therefore, when the total carbon equivalent of $CO_2$ contained in the top gas introduced into the distribution process is set to 1, the carbon equivalent of $CO_2$ introduced into the reforming process is represented by the following expression.

$$1 - Y + Y(1 - \eta) = 1 - Y\eta$$

Then, for example, when X = 1, that is, the ratio of the amount of $H_2$ and CO consumed in the reduction process is 1:1, 100 % progress of the reaction in Expression (iii) produces a reducing gas with $H_2$:CO = 1:1, which can compensate for $H_2$ and CO equivalent to the amounts consumed in the reduction process. In this case, it is necessary to supply the reforming process with an amount of $CO_2$ equivalent to the $CH_4$ contained in the regenerative methane gas.
$Y\eta$: 1 - $Y\eta$ = 1:1

Transforming this,

$$Y = 1/(2\eta).$$

That is, when X = 1, $Y_0 = 1/(2\eta)$.

Even when X < 1, that is, the amount of CO consumed in the reduction process is greater than the amount of $H_2$, it is optimal to allow 100 % of the reaction in Expression (iii) to proceed in order to maintain the mass balance. Therefore, $Y_0 = 1/(2\eta)$ in this case as well.

On the other hand, as the value of X increases, the ratio of the $H_2O$ reforming reaction in Expression (iv) needs to be increased. In other words, it is necessary to increase the proportion of $CO_2$ introduced into the synthesis process with respect to the $CO_2$ contained in the top gas by increasing the distribution ratio Y to the first top gas in the distribution process.

Here, when Y = 1,
of the reforming reactions in the reforming process, the ratio of the $CO_2$ reforming reaction in Expression (iii) is represented by

$$1 - \eta \ (\times 100 \ (\%)).$$

The ratio of the $H_2O$ reforming reaction in Expression (iv) is represented by

$$\eta - (1 - \eta) = 2\eta - 1 \ (\times 100 \ (\%)).$$

Therefore, the $H_2$/CO ratio of the reducing gas obtained in the reforming process is represented by the following expression.

$$\{2 \times (1 - \eta) + 3(2\eta - 1)\} \ / \ \{2 \times (1 - \eta) + 2\eta - 1\} = 4\eta - 1$$

Therefore, when X = $4\eta$ - 1, Y = 1, that is, all of the top gas is distributed as the first top gas in the distribution process, and the reforming reaction in Expression (iv) proceeding as much as possible according to $\eta$ is optimal to maintain the mass balance. Therefore, $Y_0 = 1$ in this case.

Even when X > $4\eta$ - 1, that is, when the consumed $H_2$/CO in the reduction process exceeds $4\eta$ - 1, it is optimal to allow the reaction in Expression (iv) to proceed as much as possible in order to maintain the mass balance. Therefore, $Y_0 = 1$ in this case.

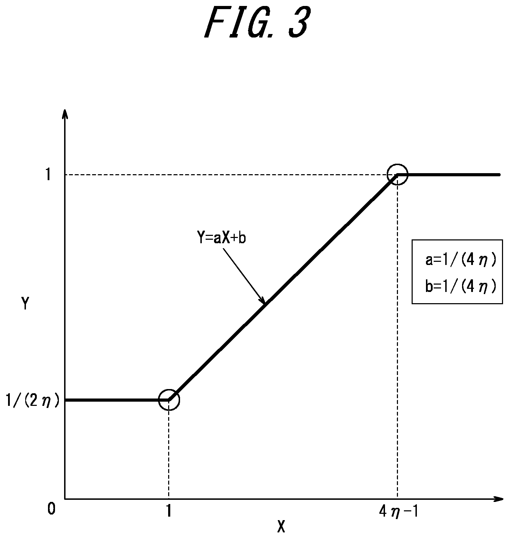

Further, when 1 < X < $4\eta$ - 1, the reference distribution ratio $Y_0$ to the first top gas is, as illustrated in FIG. 3, a straight line $Y_0 = aX + b$ connecting two points with the threshold values $Y_0 = 1/(2\eta)$ when X = 1 and $Y_0 = 1$ when X = $4\eta$ - 1. Here, a and b are as follows.

$$a = 1/(4\eta)$$

$$b = 1/(4\eta)$$

That is, when $1 < X < 4\eta - 1$, $Y_0 = (X + 1)/(4\eta)$.

The distribution ratio of $Y \times 100$ (%) to the first top gas is controlled around the standard distribution ratio of $Y_0 \times 100$ (%), which is calculated according to X. For example, $Y \times 100$ (%) is preferably controlled in the range of $Y_0 \times 100$ (%) $\pm 5$ %. $Y \times 100$ (%) is more preferably in the range of $Y_0 \times 100$ (%) $\pm 3$ %. $Y \times 100$ (%) is even more preferably in the range of $Y_0 \times 100$ (%) $\pm 1$%. The distribution ratio of $Y \times 100$ (%) to the first top gas is most preferably controlled to $Y_0 \times 100$ (%). This allows the gas balance of the circulation system to be controlled by maintaining the $H_2/CO$ of the reducing gas within a certain range without significant fluctuations.

When $X < 1$, even when the reaction in Expression (iii) proceeds 100 %, then an excess of $H_2$ (hereinafter also referred to as excess $H_2$) is generated in the circulation system. Therefore, in this case, excess $H_2$ is preferably recovered from the circulation system. The process for recovering excess $H_2$ may be, for example, immediately before the reforming process or after the reforming process and before the blowing process. The amount of excess $H_2$ recovered is preferably determined so that the amount of $H_2$ contained in the reducing gas blown into the reduction furnace is kept constant and the reaction in Expression (iii) proceeds 100 %. Therefore, in view of the above, the process of recovering excess $H_2$ is preferably after the reforming process and before the blowing process.

Further, in the case of $X > 4\eta - 1$, even when the reaction in Expression (iv) proceeds 100 %, there will be a lack of $H_2$ in the circulation system. Therefore, in this case, additional $H_2$ is preferably supplied to the circulation system. The amount of additional $H_2$ supplied is preferably determined so that the amount of $H_2$ in the reducing gas blown into the reduction furnace is kept constant. Therefore, the process of additionally supplying $H_2$ is preferably, for example, immediately before the reforming process or after the reforming process and before the blowing process.

When the distribution ratio of Y to the first top gas does not match the standard distribution ratio of $Y_0$, the consumed $H_2/CO$ and the $H_2/CO$ of the reducing gas obtained in the reforming process do not exactly match. However, as mentioned above, it is acceptable when the distribution ratio of $Y \times 100$ (%) to the first top gas is within the range of $Y_0 \times 100 \pm 5$ %. In this case, for example, $H_2$ is preferably supplied or recovered as appropriate to keep the amount of $H_2$ in the reducing gas constant.

Here, we also focus on the mass balance of the $CO_2$ cycle. That is, CO and $H_2$ are assumed to be unchanged before and after the synthesis process and the reforming processes, and therefore when the methanation reaction of CO according to Expression (ii) occurs in the synthesis process, the $H_2/CO$ of the reducing gas changes. Therefore, as described above, it is preferable to have a separation process to separate the first top gas into carbon dioxide gas and remaining gas, and to use the carbon dioxide gas obtained in the separation process as the first top gas in the synthesis process. Further, a higher $CO_2$ concentration in the carbon dioxide gas is preferred, preferably 90 vol% or more. The $CO_2$ concentration in the carbon dioxide gas is optimally 100 vol%. When methanation of CO occurs in the synthesis process, for example, $H_2$ may be supplied or recovered as appropriate to keep the amount of $H_2$ in the reducing gas constant.

The distribution ratio to the first top gas in the distribution process may be constant or may be changed at any time. For example, the components of the reducing gas and the top gas can be measured and the distribution ratio to the first top gas in the distribution process can be changed according to the variation of X: consumed $H_2/CO$. Further, the distribution ratio to the first top gas at the time of apparatus startup may be determined, for example, from past operation history, and then the distribution ratio to the first top gas may be controlled as appropriate according to the variation of X: consumed $H_2/CO$.

Further, in the method of producing reduced iron according to an embodiment of the present disclosure, the flow rate of the reducing gas supplied to the reduction furnace is preferably 1500 $Nm^3/t$ or more. The flow rate is preferably 3500 $Nm^3/t$ or less. That is, when the flow rate of the reducing gas supplied to the reduction furnace is too low, production decreases and product properties degrade. On the other hand, when the flow rate of the reducing gas supplied to the reduction furnace is excessive, gas flow resistance in the reduction furnace increases and the raw material (pellet) in the reduction furnace does not descend. Therefore, the flow rate of the reducing gas supplied to the reduction furnace is preferably 1500 $Nm^3/t$ or more. The flow rate is preferably 3500 $Nm^3/t$ or less.

Further, the iron oxide raw material used in the method of producing reduced iron according to an embodiment of the present disclosure is, for example, iron ore. Examples include lumped iron ore (lump ore), pellets (powdered iron ore hardened into a spherical shape), and the like. The grade of iron ore used as iron oxide raw material, that is, the iron content, is not particularly limited. From the perspective of reduction in a shaft furnace, the iron content is typically preferably 65 mass% or more. However, in recent years, prices are expected to soar due to the depletion of high-grade iron ore from South America and other sources. Therefore, low-grade iron ore that has an iron content of 63 mass% or less is preferably used as required. Such low-grade iron ore is inexpensive and abundant, and places of origin include, for example, Australia.

In addition, the method of producing reduced iron according to an embodiment of the present disclosure describes, among other things, the use of a shaft furnace as a direct reduction iron-making process. However, the type of reduction furnace is not limited to this, and may be a fluidized bed, a rotary kiln, a rotary hearth furnace (RHF), or the like. Shaft furnaces are preferred as reduction furnaces because of their high production efficiency, ratio of utilization and operational stability. Further, the majority of direct reduction furnaces in operation worldwide are Midrex® and Hyl®, which are shaft furnace systems.

EXAMPLES

Examples of the present disclosure are described below.

In the circulation system illustrated in FIG. 2, reduced iron was produced according to the conditions listed in Table 1. Under all conditions, the flow rate of the reducing gas supplied to the reduction furnace was 2200 $Nm^3/t$, and the operation period was 28 days. In the distribution process, all the introduced top gas was distributed between the first top gas and the second top gas, and was not supplied to other loads. In the separation process, the first top gas was separated into carbon dioxide gas ($CO_2$ concentration: 99 vol% or more) and remaining gas, and the carbon dioxide gas was used as the first top gas in the synthesis process. Further, the remaining gas was introduced into the reforming process along with the second top gas. Both the recovery of excess $H_2$ and the additional supply of $H_2$ were carried out after the reforming process and before the blowing process. Conditions other than those described above and in Table 1 were in accordance with a conventional method. Further, the flow rates of the reducing gas and the top gas in Table 1 are rounded to the nearest integer. Therefore, the total flow rate of the reducing gas may not match the total amount of $H_2$ and CO in the reducing gas. The same is true for the top gas.

[Table 1]

Table 1

| No. | Reducing gas | | | | Top gas | | | | Methane synthesis | Distribution process | | | | Recovery of excess $H_2$ | | Additional supply of $H_2$ | | Remarks |
| --- | --- | --- | --- | --- | --- | --- | --- | --- | --- | --- | --- | --- | --- | --- | --- | --- | --- | --- |
| | Flow rate [Nm$^3$/t] | $H_2$ amount [Nm$^3$/t] | Amount of CO [Nm$^3$/t] | $H_2$/CO | Flow rate [Nm$^3$/t] | $H_2$ amount [Nm$^3$/t] | Amount of CO [Nm$^3$/t] | $H_2$/CO | $CO_2$ conversion rate η | Control of distribution ratio according to consumed $H_2$/CO ratio | X | $Y_0$ | Y | Yes or no | Volume recovered [Nm$^3$/t] | Yes or no | Additional supply [Nm$^3$/t] | Remarks |
| 1 | | 200 | 2000 | 0.10 | 1646 | 108 | 1538 | 0.07 | 0.92 | Yes | 0.20 | 0.54 | 0.54 | Yes | 370 | No | - | Example |
| 2 | | 508 | 1692 | 0.30 | 1646 | 319 | 1327 | 0.24 | 0.92 | Yes | 0.52 | 0.54 | 0.54 | Yes | 176 | No | - | Example |
| 3 | | 906 | 1294 | 0.70 | 1704 | 671 | 1033 | 0.65 | 0.90 | Yes | 0.90 | 0.56 | 0.56 | Yes | 27 | No | - | Example |
| 4 | | 1100 | 1100 | 1.00 | 1679 | 840 | 840 | 1.00 | 0.92 | Yes | 1.00 | 0.54 | 0.54 | No | - | No | - | Example |
| 5 | | 1320 | 880 | 1.50 | 1646 | 1013 | 633 | 1.60 | 0.91 | Yes | 1.24 | 0.62 | 0.62 | No | - | No | - | Example |
| 6 | | 1467 | 733 | 2.00 | 1704 | 1171 | 532 | 2.20 | 0.92 | Yes | 1.47 | 0.67 | 0.67 | No | - | No | - | Example |
| 7 | | 1533 | 667 | 2.30 | 1704 | 1231 | 473 | 2.60 | 0.90 | Yes | 1.57 | 0.71 | 0.71 | No | - | No | - | Example |
| 8 | | 1650 | 550 | 3.00 | 1646 | 1263 | 383 | 330 | 0.78 | Yes | 2.31 | 1.00 | 1.00 | No | - | No | 32 | Example |
| 9 | | 1711 | 489 | 3.50 | 1646 | 1295 | 350 | 3.70 | 0.90 | Yes | 3.00 | 1.00 | 1.00 | No | - | No | 55 | Example |
| 10 | 2200 | 1833 | 367 | 5.00 | 1679 | 1429 | 251 | 5.70 | 0.85 | Yes | 3.49 | 1.00 | 1.00 | No | - | Yes | 126 | Example |
| 11 | | 1980 | 220 | 9.00 | 1646 | 1519 | 127 | 12.00 | 0.89 | Yes | 4.93 | 1.00 | 1.00 | No | - | Yes | 222 | Example |
| 12 | | 1467 | 733 | 2.00 | 1704 | 1171 | 532 | 2.20 | 0.92 | No | 1.47 | - | - | No | - | No | - | Comparative Example |
| 13 | | 1467 | 733 | 2.00 | 1704 | 1171 | 532 | 2.20 | 0.93 | Yes | 1.47 | 0.66 | 0.70 | No | 27 | No | - | Example |
| 14 | | 1467 | 733 | 2.00 | 1704 | 1171 | 532 | 2.20 | 0.90 | Yes | 1.47 | 0.69 | 0.70 | No | 10 | No | - | Example |
| 15 | | 1467 | 733 | 2.00 | 1704 | 1171 | 532 | 2.20 | 0.90 | Yes | 1.47 | 0.69 | 0.68 | No | - | No | 4 | Example |
| 16 | | 1467 | 733 | 2.00 | 1704 | 1171 | 532 | 2.20 | 0.91 | Yes | 1.47 | 0.68 | 0.66 | No | - | No | 14 | Example |
| 17 | | 1467 | 733 | 2.00 | 1704 | 1171 | 532 | 2.20 | 0.92 | Yes | 1.47 | 0.67 | 0.63 | No | - | No | 30 | Example |
| 18 | | 1467 | 733 | 2.00 | 1704 | 1171 | 532 | 2.20 | 0.92 | Yes | 1.47 | 0.67 | 0.62 | No | - | No | 38 | Example |

EP 4 596 722 A1

In all of the Examples where the distribution ratio of the top gas in the distribution process was controlled according to the consumed $H_2$/CO, the circulation system illustrated in FIG. 2, which is extremely advantageous in realizing energy savings, that is, the system in which the top gas is circulated and reused, was stably operated with a healthy mass balance over the entire 28-day operation period. Further, $CO_2$ emissions from the circulation system could be decreased to zero.

For comparison, as No. 12, the production of reduced iron was carried out under the same conditions as No. 6, except that all of the top gas was introduced into the synthesis process without controlling the distribution ratio of the top gas. Partway through the operation period, it became impossible to maintain stable operation with a healthy mass balance, and operation had to be suspended.

REFERENCE SIGNS LIST

1     reduction furnace
1a    iron oxide
1b    reduced iron
3     deduster
4     dehydrator
5     natural gas supply
6     air supply
7     reformer
9     reducing gas blowing device
10    hydrogen supply
11    methane synthesizer
12    $CO_2$ separator
13    water vapor supply
14    top gas distributor

**Claims**

1. A method of producing reduced iron, the method comprising:

   a charging process of charging iron oxide into a reduction furnace;
   a blowing process of blowing reducing gas into the reduction furnace;
   a reduction process, in the reduction furnace, of reducing the iron oxide by the reducing gas to obtain reduced iron;
   a distribution process of distributing top gas discharged from the reduction furnace into a first top gas and a second top gas;
   a synthesis process of synthesizing regenerative methane gas from the first top gas and hydrogen gas; and
   a reforming process of using the regenerative methane gas and the second top gas as feed gas to obtain the reducing gas from the feed gas, wherein
   a distribution ratio of the top gas in the distribution process is controlled according to consumed $H_2$/CO, a ratio of an amount of $H_2$ consumed to an amount of CO consumed in the reduction process.

2. The method of producing reduced iron according to claim 1, the method further comprising a separation process between the distribution process and the synthesis process, of separating the first top gas into carbon dioxide gas and remaining gas, wherein
   the carbon dioxide gas is used as the first top gas in the synthesis process.

3. The method of producing reduced iron according to claim 1 or 2, wherein a distribution ratio, in %, of $Y \times 100$ to the first top gas in the distribution process is controlled in a range of $Y_0 \times 100 \pm 5$ %, $Y_0 \times 100$ being a standard distribution ratio, in %,
   where

   when

$$X \leq 1, \ Y_0 = 1/(2\eta)$$

when

$$1 < X < 4\eta - 1, \ Y_0 = (X + 1)/(4\eta)$$

when

$$X \geq 4\eta - 1, \ Y_0 = 1$$

and

X is consumed $H_2/CO$

$\eta \times 100$ is $CO_2$ conversion rate, in %, from methane synthesis in the synthesis process

and when the range of $Y_0 \times 100 \pm 5$ % exceeds 100 %, an upper limit of the range is 100 %.

4. The method of producing reduced iron according to claim 3, wherein the distribution ratio, in %, of $Y \times 100$ to the first top gas in the distribution process is controlled to $Y_0 \times 100$ being the standard distribution ratio, in %.

5. The method of producing reduced iron according to claim 3, wherein excess $H_2$ is recovered from a circulation system comprising the blowing process, the reduction process, the distribution process, the synthesis process, and the reforming process when the consumed $H_2/CO < 1$.

6. The method of producing reduced iron according to claim 4, wherein excess $H_2$ is recovered from a circulation system comprising the blowing process, the reduction process, the distribution process, the synthesis process, and the reforming process when the consumed $H_2/CO < 1$.

7. The method of producing reduced iron according to claim 3, wherein $H_2$ is additionally supplied to a circulation system comprising the blowing process, the reduction process, the distribution process, the synthesis process, and the reforming process when the consumed $H_2/CO > 4\eta - 1$.

8. The method of producing reduced iron according to claim 4, wherein $H_2$ is additionally supplied to a circulation system comprising the blowing process, the reduction process, the distribution process, the synthesis process, and the reforming process when the consumed $H_2/CO > 4\eta - 1$.

9. The method of producing reduced iron according to claim 1 or 2, wherein at least one of de-dusting or dehydration of the top gas is carried out prior to the distribution process.

10. The method of producing reduced iron according to claim 3, wherein at least one of de-dusting or dehydration of the top gas is carried out prior to the distribution process.

11. The method of producing reduced iron according to claim 1 or 2, wherein dehydration of the regenerative methane gas is carried out prior to the reforming process.

12. The method of producing reduced iron according to claim 3, wherein dehydration of the regenerative methane gas is carried out prior to the reforming process.

*FIG. 1*

Top gas

Natural gas

Air

Reducing gas

Discharge out of system

FIG. 2

# FIG. 3

Y=aX+b

a=1／（4 η ）
b=1／（4 η ）

## INTERNATIONAL SEARCH REPORT

<table>
<tr><td>International application No.</td></tr>
<tr><td>**PCT/JP2023/046273**</td></tr>
</table>

| A. | CLASSIFICATION OF SUBJECT MATTER |
|---|---|

*C21B 5/06*(2006.01)i; *C07C 1/04*(2006.01)i; *C07C 1/12*(2006.01)i; *C10K 3/06*(2006.01)i; *C21B 5/00*(2006.01)i; *C21B 13/02*(2006.01)i; *F27D 17/00*(2006.01)i

FI: C21B5/06; C07C1/04; C07C1/12; C10K3/06; C21B5/00 321; C21B13/02; F27D17/00 104G

According to International Patent Classification (IPC) or to both national classification and IPC

| B. | FIELDS SEARCHED |
|---|---|

Minimum documentation searched (classification system followed by classification symbols)

C21B5/06; C07C1/04; C07C1/12; C10K3/06; C21B5/00; C21B11/02; C21B13/02; F27D17/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2014-028984 A (JFE STEEL CORPORATION) 19 February 2014 (2014-02-19) paragraphs [0001]-[0033], fig. 1 | 1-2, 9, 11 |
| A | | 3-8, 10, 12 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **13 March 2024** | **26 March 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

INTERNATIONAL SEARCH REPORT
Information on patent family members

International application No.

**PCT/JP2023/046273**

| Patent document cited in search report | Publication date (day/month/year) | Patent family member(s) | Publication date (day/month/year) |
|---|---|---|---|
| JP 2014-028984 A | 19 February 2014 | (Family: none) | |

Form PCT/ISA/210 (patent family annex) (July 2022)

**REFERENCES CITED IN THE DESCRIPTION**

**Patent documents cited in the description**

- JP 2017088912 A

- JP 6190522 B